# EUROPEAN PATENT SPECIFICATION

(11) **EP 0 350 792 B1**
(45) Date of publication and mention of the grant of the patent: **23.11.1994**
(21) Application number: 89112379.6
(22) Date of filing: 06.07.1989
(51) Int. Cl.: A61B 5/14

(54) **A test blood gathering equipment**
Vorrichtung zum Sammeln von Blutproben
Dispositif pour prélever du sang à tester

(30) Priority: 13.07.1988 JP 92825/88; 29.05.1989 JP 62023/89
(43) Date of publication of application: 17.01.1990
(73) Proprietor: KAWASUMI LABORATORIES, INC., Tokyo (JP)
(72) Inventor: Minase, Chugo, Shinagawa-ku Tokyo (JP); Okubo, Yasuto, Kitakatsuragi-gun Nara-ken (JP); Yoshimura, Keiji, Sakai-shi Osaka-fu (JP)
(74) Representative: Heusler, Wolfgang, Dipl.-Ing.

(56) References cited:
- EP-A- 0 106 290
- WO-A-83/02722
- DE-A- 2 439 218
- FR-A- 2 013 819
- US-A- 4 294 247

## Description

The present invention relates to a test blood gathering equipment to be used for testing of blood such as blood crossmatch or virus test to be operated prior to blood transfusion.

For transfusing the gathered blood from a blood donor to a patient, some kinds of blood test are carried out as to whether or not the donor's blood is suitable to the patient, or whether the gathered blood is infected with virus or not.

In general, a soft plastic blood bag is used for gathering the blood from the donor. The blood bag is connected to a bendable blood introduction tube. For collecting the blood, a needle supported at the end portion of a tube is penetrated into the donor's vein. When the blood reaches up to a determined amount, the needle is drawn out from the donor and the blood introduction tube is sealed with heat at its end portion. The introduction tube is filled with the donor's blood, and for testing the blood, the heat-sealed part is cut. The tube which has been cut at the determined length and sealed at the both ends is called segment tube.
Examples of the blood crossmatch now in use with the segment tube are as follows.
(1) The segment tube is cut at its end to move the blood to a test tube.
(2) The test tube holding the blood is centrifugated at 3000 rpm and for 5 minutes to separate the blood into packed red cells and blood plasma, and the blood plasma is gathered in another test tube (the separated blood plasma is subjected to the blood crossmatch or the virus test in a manner not referred to).
   The remaining packed red cells are washed with a physiological saline salt solution, and 0.2 ml of the last remaining packed red cells are added with 9.8 ml or a salt solution to produce a resuspended red cell product of 2%.
(3) Two drops of the blood serum are taken from the patient and collected in another rest tube and one drop of the 2% resuspended red cell product is added into the blood serum.
(4) Instantly, the above material is centrifugated at 1000 to 1500 rpm for 1 minute for judging if clot of blood or hemolysis is formed, or not.
(5) Further, the above test tube is added with one drop of a 5% bromelin solution, and is left for 15 minutes at room temperatures and treated as the above step (4).
(6) In case of neither blood condensation nor blood coagulation, it is judged as suitable to the patient.

However, when the blood held in the segment tube is moved to the test tube and since its end portion is cut e.g. with scissors there is danger of virus infection as the blood contacts the operator or the scissors. As the scissors should be every time disinfected with alcohol, the operation is troublesome.

FR-A-20 13 819 already discloses a tube determined for collecting blood from a finger of a donor and is therefore equipped with sharp piercing tips at the upper end of the tube. At its lower end the tube may have an outlet passage.

It is an object of the invention to provide a test blood gathering equipment in which the segment tube may the easily opened without touching of the blood to the operator at his hand or causing dangers as the virus infection.

It is another object of the invention to provide a test blood gathering equipment in which the blood to be tested flowing from the segment tube may be moved to the test tube without touching the operator's hand.

It is a further object of the invention to provide a test blood gathering equipment in which the blood stored in the tube from the segment tube is directly subjected to the centrifugal treatment, without moving to the test tube, so as to extract the packed red cell.

Other objects and features of the invention will be apparent from following description.

These objects are achieved by the features of the claims.

When the segment tube filled with the blood is inserted in the cylindrical body, it is easily opened by the cutting means, and the blood flowing from the segment tube runs into the test tube via an opening formed at the bottom of the cylindrical body.

The blood from the segment tube may be stored in the cyclindrical body without splashing outside of the body.
Fig. 1 through Fig. 6 show embodiments of the invention, where each (a) is a cross sectional view along a vertical direction (A - A), and each (b) is a plan view;
Fig. 7 is an outlined cross sectional view showing use of the embodiment of Fig. 6;
Fig. 8 shows a further embodiment of the invention, where (a) is a cross sectional view along a vertical direction (A - A), and (b) is a plan view;
Fig. 9 and Fig.10 show still further embodiments of the invention, where each (a) is a cross sectional view along a vertical direction (A - A), and each (b) is a plan view; and
Fig.11 is an outlined view showing use of the embodiment of Fig. 9.

### DESCRIPTION OF PREFERRED EMBODIMENTS

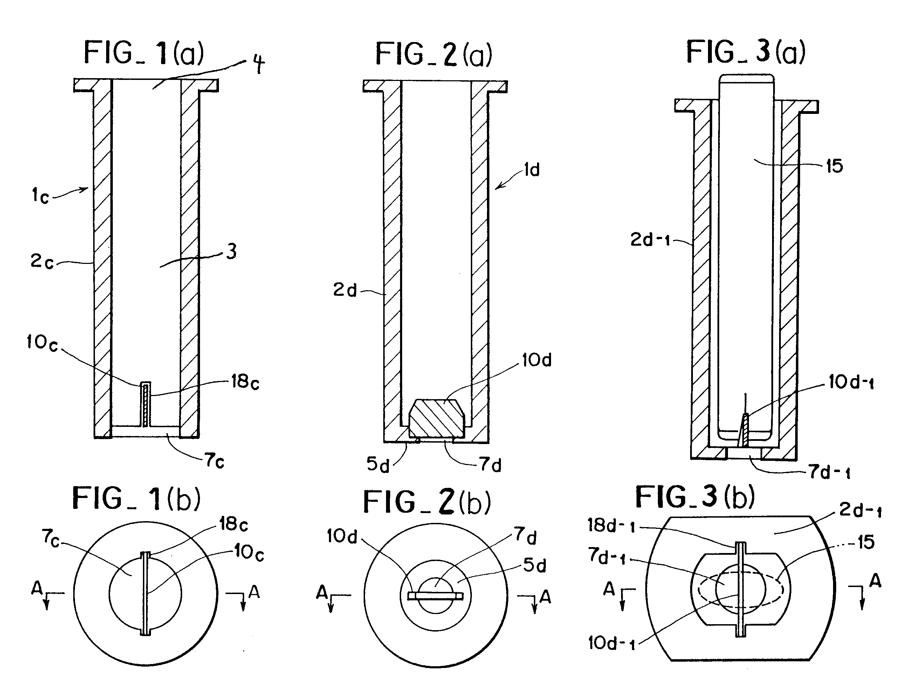

Fig. 1 shows one embodiment of the test blood gathering equipment of the invention.

The test blood gathering equipment 1c has a tube 2c made of a hard or half hard plastics with a space 3 enough to hold the segment tube containing the blood to be tested.

The tube 2c is circular in cross section of the horizontal direction but may be elliptic. It has an opening 4 at an upper part.

Test blood gathering means 1c, 1d, 1e, 1f, 1g shown in Fig. 1 to Fig. 6 have cutting pieces 10c, 10d, 10e, 10f, 10g having sharp razor edges.

The test blood gathering equipment 1c of Fig. 1 is formed with grooves 18c at the lower part of an inner wall of the tube 2c, and a cutting piece 10c is inserted thereinto. This embodiment omits a bottom, and the tube 2c opens to serve as a flowing hole 7c. The cutting piece 10c may be one part of the tube 2c.

In the test blood gathering equipment 1d of Fig. 2, the tube 2d is formed with a bottom 5d which is centrally opened with a flowing hole 7d and is secured with a cutting piece 10d.

Fig. 3 shows a modification of Fig. 2, and as is seen from (b) of the same, a horizontally cross sectional shape of the tube 2d-1 is elliptic, having a long diameter and a short diameter, in which a cutting piece 10d-1 is inserted into grooves 18d-1 at the lower part of the inner wall transversely to the long diameter.

In such a manner, when the segment tube 15 is put into the tube 2d-1 (the both end portions of the segment tube are sealed by crashing and shaped in ellipticity), the elliptic end portion of the segment tube 15 can be made to agree to the large diameter of the tube 2d-1. Since the end portion of the segment tube 15 contacts the cutting piece 10d-1 in right angle, the tube 15 can be opened exactly and easily.

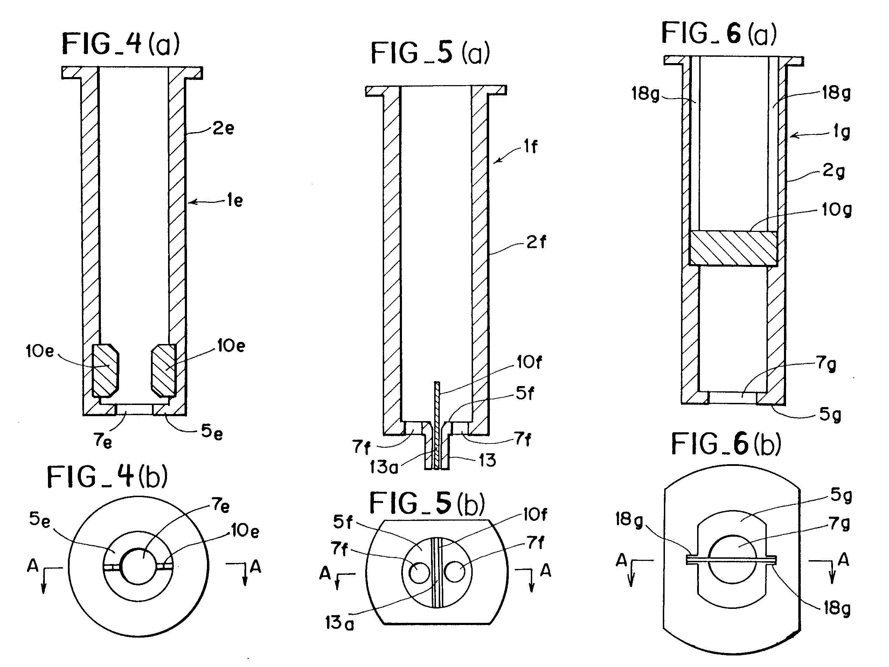

The test blood gathering equipment 1e of Fig. 4 is provided with a plurality of breaking pieces 10e, 10e at the lower part of the inner wall of the tube 2e, and is formed with a hole 7e for flowing of the blood to be tested.

The test blood gathering equipment 1f of Fig. 5 has a downward projecting attached part 13 as one part of the bottom 5f or by means of a securing means, and a cutting pierce 10f is inserted into a groove 13a formed in the attached part 13 and fixed by means of high frequency melting with heating. The bottom 5f is formed with blood flowing holes 7f symmetrically around the cutting piece 10f.

The test blood gathering equipment of Fig. 6 is attached with a cutting piece 10g at the middle part of the tube 2g. As an example of providing the cutting piece 10g, grooves 18g are formed vertically from the upper part of the inner wall to the middle part of the tube 2g, and the cutting piece 10g is inserted in the grooves 18g and secured at the middle part of the tube 2g. A bottom 5g is centrally formed with a blood flowing hole 7g.

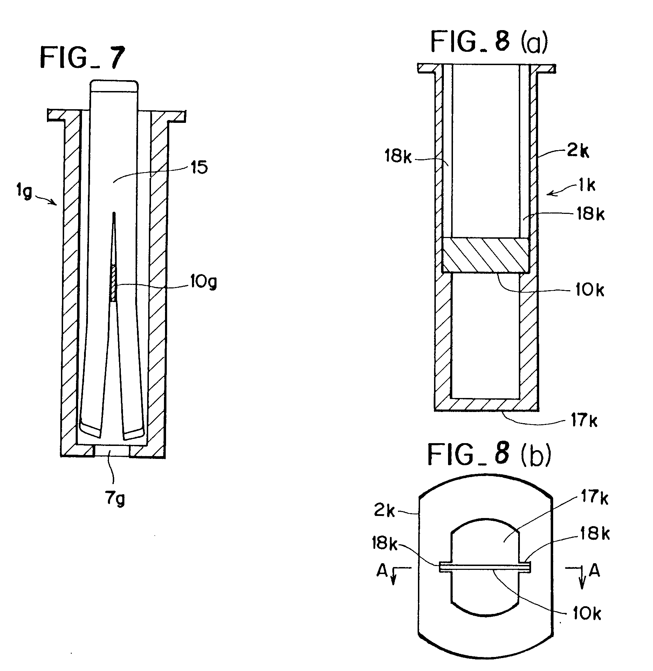

Fig. 7 shows the use of the embodiment of Fig. 6. The cutting piece 10g is provided at the middle part of the tube 2g, thereby to open the segment tube 15 vertically, so that the opened area may be enlarged. Therefore, the blood in the segment tube 15 instantly flows out of the tube 2g and drops smoothly into the test tube. If it is subjected to the centrifugal treatment, the blood is separated into the blood plasma and the packed red cells without damaging the blood components.

The test blood gathering equipment 1k in Fig. 8 is defined with grooves 18k, 18k vertically in the inner wall of a tube 2k, and a cutting piece 10k is inserted along the grooves at the middle part of the tube 2k. This tube 2k is also closed with a closing part 17k. Since the equipment 1k is provided with the cutting piece 10k at the middle part, the segment tube is broken vertically along its length as described in the embodiments of Fig. 6 and Fig. 7 and as the opened area is made large, the blood to be tested in the segment tube can flow into the test tube 2k smoothly. The blood stored in the tube 2k is moved to the test tube for blood testings.

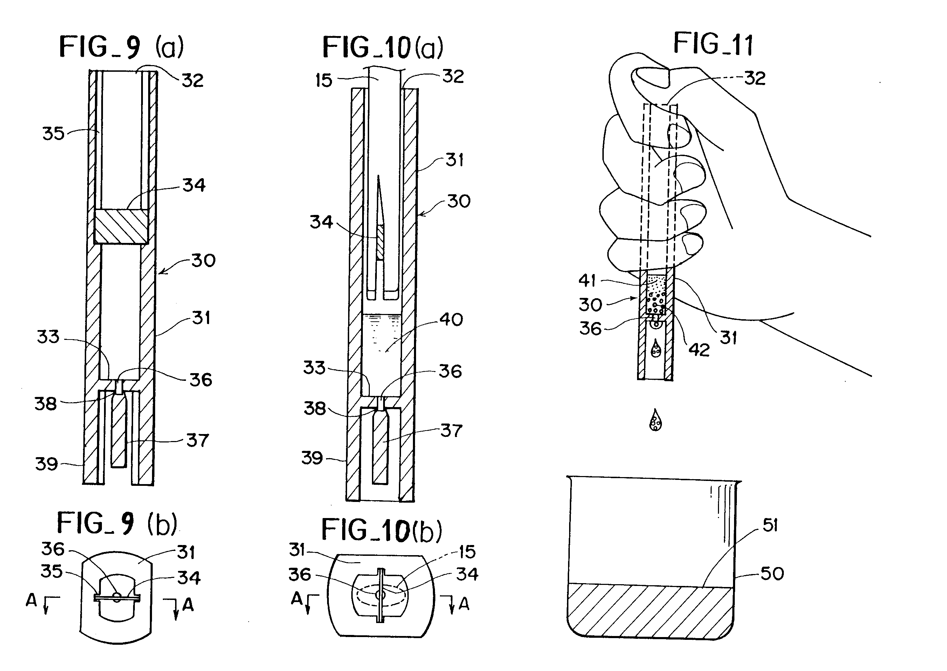

Fig. 9 shows a further embodiment of the invention.

In the test blood gathering equipment 30, the tube 31 of a hard or half hard plastic material is opened at the upper part 32, and is defined with a flowing hole 36 centrally of a bottom 33. The bottom 33 is provided with a closing piece 37 of the flowing hole 36 integrally with the bottom 33, and when the closing piece 37 is broken at a thin part 38, the flowing hole 36 is opened.

The closing piece 37 is formed with a plurality of wings 39 at its outer circumference, which prevent the closing piece 37 from breaking by contacting the circumferential wall during the centrifugal treatment of the test blood gathering equipment 30.

The closing piece 37 and the wings 39 may be formed as parts of the tube 31, or may be independent of the latter and fixed thereto with such as adhesive.

The cutting piece 34 is furnished at the middle part of the tube 31, and an attaching means of the piece 34 is inserted along the grooves 35.

The shown tube 31 is elliptic in the horizontal cross section but may be circular.

Fig.10 shows use of the test blood gathering equipment 30. If the segment tube 15 is pressed down into the tube 31, it is broken vertically by means of the cutting piece 34, and the blood to be tested flows out into the tube 31. The segment tube 15 is drawn out from the tube, and the equipemnt 30 is set on the centrifugal separator for separating the blood 40 into the blood plasma 41 and the packed red cell 42 (see Fig.11). It is not necessary that the segment tube 15 is urged into the tube 31, but the former naturally goes into the latter due to the centrifugal force as said above.

The upper opening 32 of the tube 31 is closed with a thumb as shown in Fig.11, and the closing piece 37 is effected with an external force by, for example, a pincette, and a thin part 38 is broken to form a flowing hole 36. Subsequently, the opening 32 is released by gradually moving the thumb, and the packed red cells 42 at the lower part of the tube 31 drop from the flowing hole 36 into the test tube 50 storing the patient's blood 51 for carrying out the blood crossmatch.

If using the test blood gathering equipment shown in Fig. 9, the process of setting the equipment to the test tube may be omitted, and the blood stored in the tube 31 may be directly subjected to the centrifugal seperation.

In dependence upon the test blood gathering equipment of the invention, since the segment tube is inserted into the cylindrical tube and is opened by the cutting means provided within the cylindrical tube, the operation is more easier than the foregoing manner which opens the segment tube by means of scissors, and has no danger of virus infection because the blood to is not touched by the operator at his hand.

Further, if the present equipment is attached with the same number as an identification number of the segment tube, the blood may be prevented from mis-dealing and other human errors.

## Claims

1. A test blood gathering equipment for opening a segment tube (15) which holds blood to be tested and for getting the blood out of the tube (15), characterized by
a structure comprising a generally cylindrical body (2c - 2k; 31) having at its upper part an opening (4) for inserting the segment tube (15) thereinto, and a blood flowing hole (7c - 7g; 36) at its lower part, and
a razor edge-shaped cutting piece (10c - 10k; 34) secured to an inner wall of said cylindrical body (2c - 2k; 31),
said cutting piece (10c - 10k; 34) extending in a direction so as to open the segment tube (15) by means of said cutting piece (10c - 10k; 34) when the tube (15) is inserted into said body (2c - 2k; 31).

2. The equipment as claimed in claim 1, characterized by said razor edge-shaped cutting piece (10g) being secured to the inner wall of said cylindrical body (2g) at a middle part thereof.

3. The equipment as claimed in claim 1, characterized by said razor edge-shaped cutting piece (10c - 10f) being secured to the inner wall of said cylindrical body (2c - 2f) at a lower end part or a bottom part thereof.

4. The equipment as claimed in any one of claims 1 to 3, characterized by said razor edge-shaped cutting piece (10c, 10d-1, 10f - 10k, 34) traversing the cylindrical body (2c, 2d-1, 2f - 2k, 31) along the whole diameter thereof.

5. The equipment as claimed in any one of claims 1 to 4, characterized by said razor edge-shaped cutting piece (10c, 10d-1, 10g, 10k, 34) being secured at both ends of the inner wall of said cylindrical body (2c, 2d-1, 2g, 2k, 31).

6. The equipment as claimed in any one of the preceding claims, characterized by a horizontal cross-section of said cylindrical body (2d-1, 31) being elliptic, and said razor edge-shaped cutting piece (10d-1, 31) being provided in a direction crossing the large diameter of the ellipse.

7. The equipment as claimed in any one of claims 1 to 6, characterized by a structure closing the blood flowing hole (36) of said cylindrical body (31) with a sealing member (37), said sealing member (37) being adapted to be broken at a thin part (38) for opening said blood flowing hole (36).

## Patentansprüche

1. Testblutsammelausrüstung zum Öffnen eines Segmentschlauchs (15), welcher zu testendes Blut enthält, und zum Herausbringen des Bluts aus dem Schlauch (15), gekennzeichnet durch
eine Struktur mit einem allgemein zylindrischen Körper (2c - 2k; 31), der an seinem oberen Teil eine Öffnung (4) zum Einsetzen des Segmentschlauchs (15) dorthinein, sowie eine Blutströmungsöffnung (7c - 7g; 36) an seinem unteren Teil aufweist, und
ein rasierklingenförmiges Schneidstück (10c - 10k; 34), das an einer Innenwand des zylindrischen Körpers (2c - 2k; 31) befestigt ist,
wobei das Schneidstück (10c - 10k; 34) sich in einer Richtung erstreckt, daß es den Segmentschlauch (15) mittels des Schneidstücks (10c - 10k; 34) öffnet, wenn der Schlauch (15) in den Körper (2c - 2k; 31) eingesetzt wird.

2. Ausrüstung nach Anspruch 1, dadurch gekennzeichnet, daß das rasierklingenförmige Schneidstück (10g) an der Innenwand des zylindrischen Körpers (2g) an einem mittleren Teil desselben befestigt ist.

3. Ausrüstung nach Anspruch 1, dadurch gekennzeichnet, daß das rasierklingenförmige Schneidstück (10c - 10f) an der Innenwand des zylindrischen Körpers (2c - 2f) an einem unteren Endteil oder einem Bodenteil desselben befestigt ist.

4. Ausrüstung nach einem der Ansprüche 1 bis 3, dadurch gekennzeichnet, daß das rasierklingenförmige Schneidstück (10c, 10d-1, 10f - 10k, 34) den zylindrischen Körper (2c, 2d-1, 2f - 2k, 31) längs des gesamten Durchmessers desselben quert.

5. Ausrüstung nach einem der Ansprüche 1 bis 4, dadurch gekennzeichnet, daß das rasierklingenförmige Schneidstück (10c, 10d-1, 10g, 10k, 34) an beiden Enden der Innenwand des zylindrischen Körpers (2c, 2d-1, 2g, 2k, 31) befestigt ist.

6. Ausrüstung nach einem der vorhergehenden Ansprüche, dadurch gekennzeichnet, daß der horizontale Querschnitt des zylindrischen Körpers (2d-1, 31) elliptisch ist, und das rasierklingenförmige Schneidstück (10d-1, 31) in einer den großen Durchmesser der Ellipse kreuzenden Richtung vorgesehen ist.

7. Ausrüstung nach einem der Ansprüche 1 bis 6, gekennzeichnet durch eine die Blutströmungsöffnung (36) des zylindrischen Körpers (31) mit einem abdichtenden Element (37) verschließende Struktur, wobei das abdichtende Element (37) so ausgebildet ist, daß es zum Öffnen der Blutströmungsöffnung (36) an einem dünnen Teil (38) zerbrochen wird.

## Revendications

1. Equipement de prélèvement de sang pour un test, destiné à ouvrir un tube segment (15) qui contient du sang à tester et pour faire s'écouler le sang à l'extérieur du tube (15), caractérisé en ce qu'il comprend une structure comportant un corps généralement cylindrique (2c-2k; 31) présentant, à sa partie supérieure, une ouverture (4) pour l'insertion du tube segment (15) dans ce corps, et, à sa partie inférieure, un trou (7c-7g;36) pour l'écoulement du sang, et une pièce de coupe en forme de lame de rasoir (10c-10k;34) fixée à une paroi interne du corps cylindrique (2c-2k;31), cette pièce de coupe (10c-10k;34) s'étendant dans une direction telle que l'ouverture du tube segment (15) ait lieu au moyen de cette pièce de coupe (10c-10k;34) lorsque le tube (15) est introduit dans le corps (2c-2k;31).

2. Equipement suivant la revendication 1 caractérisé en ce que la pièce de coupe en forme de lame de rasoir (10g) est fixée à la paroi interne du corps cylindrique (2g), dans sa partie médiane.

3. Equipement suivant la revendication 1 caractérisé en ce que la pièce de coupe en forme de lame de rasoir (10c-10f) est fixée à la paroi interne du corps cylindrique (2c-2f) à l'endroit d'une partie extrême inférieure ou à une partie inférieure de ce corps.

4. Equipement suivant l'une quelconque des revendications 1 à 3 caractérisé en ce que la pièce de coupe en forme de lame de rasoir (10c,10d-1, 10f -10k, 34) traverse le corps cylindrique (2c, 2d-1,2f - 2k, 31) le long de la totalité de son diamètre.

5. Equipement suivant l'une quelconque des revendications 1 à 4 caractérisé en ce que la pièce de coupe en forme de lame de rasoir (10c,10d-1, 10g, 10k,34) est fixée, à ces deux extrémités, à la paroi interne du corps cylindrique (2c,2d-1,2g,2k,31).

6. Equipement suivant l'une quelconque des revendications précédentes caractérisé en ce que la section transversale horizontale du corps cylindrique (2d-1,31) est elliptique et la pièce de coupe en forme de lame de rasoir (10d-1,31) s'étend dans une direction croisant le grand diamètre de l'ellipse.

7. Equipement suivant l'une quelconque des revendications 1 à 6 caractérisé en ce qu'il comprend une structure fermant le trou (36) d'écoulement du sang du corps cylindrique (31) au moyen d'un organe d'obturation (37), cet organe d'obturation (37) étant adapté de manière à être arraché, à l'endroit d'une partie mince (38), pour ouvrir le trou d'écoulement du sang (36).
